(19) Europäisches Patentamt
European Patent Office
Office européen des brevets

(11) EP 1 907 526 B1

(12) EUROPEAN PATENT SPECIFICATION

(45) Date of publication and mention of the grant of the patent:
**14.05.2014 Bulletin 2014/20**

(21) Application number: **06762810.7**

(22) Date of filing: **25.07.2006**

(51) Int Cl.:
***C11D 17/04*** *(2006.01)* ***C11D 3/37*** *(2006.01)*
***A61K 8/02*** *(2006.01)* ***A61Q 11/02*** *(2006.01)*
***A61Q 19/10*** *(2006.01)* ***A61K 8/81*** *(2006.01)*
***A61Q 1/14*** *(2006.01)*

(86) International application number:
**PCT/EP2006/007324**

(87) International publication number:
**WO 2007/017098 (15.02.2007 Gazette 2007/07)**

(54) **PROCESS FOR REMOVING DIRT OR MAKE-UP FROM SURFACES**

VERFAHREN ZUM ENTFERNEN VON SCHMUTZ ODER MAKE-UP VON OBERFLÄCHEN

PROCEDE D ELIMINATION DE SALETES OU DE MAQUILLAGE SUR DES SURFACES

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.07.2005 US 701975 P**

(43) Date of publication of application:
**09.04.2008 Bulletin 2008/15**

(73) Proprietor: **RHODIA CHIMIE**
**93300 Aubervilliers (FR)**

(72) Inventor: **AUBAY, Eric**
**F-92100 Clichy (FR)**

(74) Representative: **Jacobson, Claude et al**
**Cabinet Lavoix**
**2, Place d'Estienne d'Orves**
**75441 Paris Cedex 09 (FR)**

(56) References cited:
**EP-A- 1 066 825**
**DE-A1- 10 062 355**
**US-A- 4 784 789**
**US-A1- 2005 124 519**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

BACKGROUND OF THEN INVENTION

The invention concerns the use of an agent for transferring dirt or make-up from surfaces, in a process for removing dirt or make-up from surfaces, such as domestic, institutional or body surfaces (keratinous surfaces such as skin or hair, or teeth surfaces) typically carried out when performing cleaning.

Cleaning of surfaces, for example hard surfaces or body surfaces, is an operation that is performed on a regular on non regular basis, for the sake of hygiene and/or comfort, and/or perception of hygiene or comfort. In cleansing, removing dirt from surfaces is one embodiment. Presence of dirt is easily perceived by people, who can feel a need for having it removed. Removal of dirt can be easily perceived by the person performing the cleaning.

The industry provides all sorts of means and compositions for performing cleaning and removal of dirt. The means and compositions are used by consumers in private places, when performing private house-hold, or by people performing institutional house-hold operations in public places, such as janitors and maids.

There is a need for improving dirt removal or perception of dirt removal, and/or for improving hygiene, comfort, and/or perception thereof. There is also a need for improving the efficiency of dirt removal (more dirt removed and/or same amount but quicker or with less efforts) or the perception thereof.

There is also a need for removing dirt or cosmetics such as make-up from body surfaces (keratinous surfaces such as skin or hair, or teeth surfaces). In polluted areas, particles deposit onto the hair and/or skin. They are to be removed or cleaned. Make-up can also be considered as dirt when it is to be removed. Also there is a need for removing sebum secretions or greasy substances from hair and/or skin.

There is also a need for removing dirt from plants.

Document US 4 784 789 discloses a method in removing soils from hard surfaces comprising applying an aqueous composition including a polymer an the soil.

BRIEF SUMMARY OF THE INVENTION

The invention provides the use of an agent for transferring dirt or make-up from surfaces, in a process that addresses at least some of the needs mentioned above.

Thus the invention concerns the use, as an agent for transferring dirt or make-up from surfaces, of an ampholytic or zwitterionic polymer, in a process for removing dirt or make-up form surfaces, comprising the steps of

a) Applying a fluid composition comprising an ampholytic or zwitterionic polymer onto the surface having dirt or make-up, by pouring or spraying the composition onto the surface or by using an application mean,
b) simultaneously or subsequently spreading and/or wiping the composition onto the surface, with a wiping or spreading fibrous or porous mean, and then,
c) optionally removing the liquid, or a part thereof, from the surface with a drying fibrous or porous mean,

some dirt or make-up being transferred from the surface to the application mean and/or the wiping mean and/or the spreading mean, and/or the drying mean

The invention also concerns the use of the ampholytic or zwitterionic polymer for removing dirt or make-up, or improving dirt or make-up removal In a particular embodiment it concerns the use of an ampholytic or zwitterionic polymer in a liquid composition for removing dirt from a domestic or institutional surface, for improving dirt removal It also concerns the use of the liquid composition comprising the polymer in the process for removing dirt or make-up form surfaces.

The treated surface can be for example

- a domestic or institutional hard surface,
- a tooth,
- a keratinous surface such as skin or hair, or
- a plant.

Without intending to be bound to any theory, it is believed that the ampholytic or zwitterionic polymer provides some interaction between the dirt and the application mean and/or the wiping mean and/or the spreading mean, and/or the drying mean, especially when theses means comprise a cellulosic material, said interaction helping in transferring the dirt from the surface to these means.

DETAILED DESCRIPTION OF THE INVENTION

Definitions

In the present specification "composition comprising **a** polymer" refers to a composition that can be used as such, or to a composition that is to be diluted before or during use (it can be referred to a "concentrate composition") or to a composition in a diluted form (it can be referred to a "diluted composition")

In the present specification "hard surface" refers to any surface found in homes or institutional buildings, or in industrial buildings, or in furniture, provided that the surface is not a fabric such as clothes, furniture fabrics, curtains, sheets

The dirt or make-up transfer index is $DeltaE = \sqrt{DL^2 + Da^2 + Db^2}$

wherein.

DL = L before the process - L after the process
Da = a before the process - a after the process
Db = b before the process - b after the process L

a, and b are colorimetric CIE L*a*b coordinates for example measure with a LU600 version 010892 type apparatus, of the surface before and after removal of dirt or make-up, or of the application mean and/or the wiping mean and/or the spreading mean, and/or the drying mean, before and after removal of dirt or make-up

Domestic surfaces are understood as surfaces found in private buildings or parts of buildings such as houses and apartments, that are to be cleaned on a regular or not basis

Institutional surfaces are understood as surfaces found in public buildings, such as work places, hotels, schools, administrations, entertainment facilities, that are to be cleaned on a regular or not basis

In the present application, the expression "unit derived from a monomer" denotes a unit that can be obtained directly from said monomer by polymerization. Thus, for example, a unit derived from an acrylic or methacrylic acid ester does not cover a unit of formula $-CH_2-CH(COOH)-$, $-CH_2-C(CH_3)(COOH)-$, $-CH_2-CH(OH)-$, respectively, obtained for example by polymerizing an acrylic or methacrylic acid ester, or vinyl acetate, respectively, and then hydrolyzing. A unit denved from acrylic or methacrylic acid covers, for example, a unit obtained by polymerizing a monomer (for example an acrylic or methacrylic acid ester), and then reacting (for example by hydrolysis) the polymer obtained so as to obtain units of formula $-CH_2-CH(COOH)-$, or $-CH_2-C(CH_3)(COOH)-$. A unit derived from a vinyl alcohol covers, for example, a unit obtained by polymerizing a monomer (for example a vinyl ester), and then by reacting (for example by hydrolysis) the polymer obtained so as to obtain units of formula $-CH_2-CH(OH)-$.

In the present application, unless otherwise mentioned, the average molar masses are number-average molar masses, measured by stearic exclusion chromatography in an appropnate solvent, coupled to a multiangle light scattenng detector (GPC-MALLS). In the present application, reference may also be made to theoretical average molar masses, determined from the masses of constituents used to prepare the polymers

In the present application, the term "hydrophobic" is used in its usual sense of "that which has no affinity for water", this means that the organic polymer of which it consists, taken alone, (of the same composition and of the same molar mass), would form a two-phase macroscopic solution in distilled water at 25°C, at a concentration of greater than 1% by weight.

In the present application, the term "hydrophilic" is also used in its usual sense of "that which has affinity for water", i.e. that which is not capable of forming a two-phase macroscopic solution in distilled water at 25°C at a concentration of greater than 1% by weight

Polymer

The ampholytic polymers comprise several units. Thus it is a copolymer Copolymers are understood as polymers having several different units, as opposed to homopolymers Copolymers include terpolymers The zwitterionic polymers can be homopolymers or copolymers The copolymers are preferably random copolymers According to an advantageous embodiment, the copolymer is water-soluble soluble or water-dispersible. This means that said copolymer does not, over at least a certain pH and concentration range, form a two-phase composition in water; under the conditions of use

The ampholytic polymers typically comprise.

- cationic or potentially cationic units C,
- anionic or potentially anionic units A, and
- optionally non ionic, hydrophilic or hydrophobic units N

The zwitterionic polymers typically comprise:

- zwitterionic units Z, bearing a cationic charge and an anionic charge on the same unit,
- optionally other units, being anionic or potentially anionic units, or non ionic hydrophilic or hydrophobic units

The expression "cationic or potentially cationic units C" is intended to mean units that comprise a cationic or potentially cationic group The cationic units or groups are units or groups that have at least one positive charge (generally associated with one or more anions such as the chloride ion, the bromide ion, a sulfate group, a methyl sulfate group), whatever the pH of the medium in which the copolymer is present The potentially cationic units or groups are units or groups that may be neutral or may have at least one positive charge according to the pH of the medium in which the copolymer is present. In this case, reference will be made to potentially cationic units C in neutral form or in cationic form By extension, reference may be made to cationic or potentially cationic monomers

The expression "anionic or potentially anionic units A" is intended to mean units that comprise an anionic or potentially anionic group The anionic units or groups are units or groups that have at least one negative charge (generally associated with one or more cations such as cations of alkali metal or alkaline earth metal, for example sodium, compounds, or cationic groups such as ammonium), whatever the pH of the medium in which the copolymer is present The potentially anionic units or groups are units or groups that may be neutral or may have at least one negative charge according to the pH of the medium in which the copolymer is present. In this case, reference will be made to potentially anionic units A in neutral form or in anionic form By extension, reference may be made to anionic or potentially anionic monomers

The term "neutral units N" is intended to mean units that have no charge, whatever the pH of the medium in which the copolymer is present

Examples of <u>potentially cationic monomers</u> (from which potentially cationic units can derive), include

- N,N-(dialkylamino-ω-alkyl)amides of α,β-monoethylenically unsaturated carboxylic acids, such as N,N-dimethylaminomethylacrylamide or -methacrylamide , 2-(N,N-dimethylamino)ethylacrylamide or -methacrylamide, 3-(N,N-dimethylamino)propylacrylamide or methacrylamide, and 4-(N,N-dimethylamino)butylacrylamide or -methacrylamide,
- α,β-monoethylenically unsaturated amino esters such as 2-(dimethylamino)ethyl acrylate (DMAA), 2-(dimethylamino)ethyl methacrylate (DMAM), 3-(dimethylamino)propyl methacrylate, 2-(tert-butylamino)ethyl methacrylate, 2-(dipentylamino)ethyl methacrylate, and 2(diethylamino)ethyl methacrylate,
- vinylpyridines,
- vinylamine,
- vinylimidazolines,
- monomers that are precursors of amine functions such as N-vinylformamide, N-vinylacetamide, etc., which give nse to primary amine functions by simple acid or base hydrolysis

Examples of <u>cationic monomers</u> (from which cationic units can derive) include:

- acryloyl- or acryloyloxyammonium monomers such as trimethylammonium propyl methacrylate chloride, trimethylammonium ethylacrylamide or -methacrylamide chloride or bromide, trimethylammonium butylacrylamide or -methacrylamide methyl sulfate, trimethylammonium propylmethacrylamide methyl sulfate, (3-methacrylamidopropyl)trimethylammonium chloride (MAPTAC), (3-methacrylamidopropyl)trimethylammonium methyl sulphate (MAPTAMES), (3-acrylamidopropyl)trimethylammonium chloride (APTAC), methacryloyloxyethyl-trimethylammonium chloride or methyl sulfate, and acryloyloxyethyltrimethylammonium chloride,
- 1-ethyl-2-vinylpyridinium or 1-ethyl-4-vinylpyridinium bromide, chloride or methyl sulfate;
- N,N-dialkyldiallylamine monomers such as N,N-dimethyldiallylammonium chloride (DADMAC);
- polyquaternary monomers such as dimethylaminopropylmethacrylamide chloride and N-(3-chloro-2-hydroxypropyl)trimethylammonium (DIQUAT), etc

Examples of <u>anionic or potentially anionic monomers</u> (from which anionic or potentially anionic units can denve) include:

- monomers having at least one carboxylic function, for instance α,β-ethylenically unsaturated carboxylic acids or the corresponding anhydrides, such as acrylic, methacrylic or maleic acids or anhydrides, fumaric acid, itaconic acid, N-methacroylalanine, N-acryloylglycine, and their water-soluble salts,
- monomers that are precursors of carboxylate functions, such as tert-butyl acrylate, which, after polymerization, give rise to carboxylic functions by hydrolysis,

- monomers having at least one sulfate or sulfonate function, such as 2-sulfooxyethyl methacrylate, vinylbenzene sulfonic acid, allyl sulfonic acid, 2-acrylamido-2-methylpropane sulfonic acid, sulfoethyl acrylate or methacrylate, sulfopropyl acrylate or methacrylate, and their water-soluble salts,
- monomers having at least one phosphonate or phosphate function, such as vinylphosphonic acid, etc , the esters of ethylenically unsaturated phosphates, such as the phosphates derived from hydroxyethyl methacrylate (Empicryl 6835 from Rhodia) and those derived from polyoxyalkylene methacrylates, and their water-soluble salts

Examples of hydrophobic non ionic monomers (from which hydrophobic non ionic units can derive) include:

- vinylaromatic monomers such as styrene, alpha-methylstyrene, vinyltoluene, etc ,
- vinyl halides or vinylidene halides, such as vinyl chloride, vinylidene chloride,
- $C_1$-$C_{12}$ alkylesters of $\alpha,\beta$-monoethylenically unsaturated acids such as methyl, ethyl or butyl acrylates and methacrylates, 2-ethylhexyl acrylate, etc.,
- vinyl esters or allyl esters of saturated carboxylic acids, such as vinyl or allyl acetates, propionates, versatates, stearates, etc ,
- $\alpha,\beta$-monoethylenically unsaturated nitriles containing from 3 to 12 carbon atoms, such as acrylonitrile, methacrylonitrile, etc.,
- $\alpha$-olefins such as ethylene, etc.,
- conjugated dienes, such as butadiene, isoprene, chloroprene

Examples of non ionic hydrophilic monomers (from which hydrophilic non ionic units can derive), include:

- hydroxyalkyl esters of $\alpha,\beta$-ethylenically unsaturated acids, such as hydroxyethyl or hydroxypropyl acrylates and methacrylates, glyceryl monomethacrylate, etc ,
- $\alpha,\beta$-ethylenically unsaturated amides such as acrylamide, N,N-dimethylmethacrylamide, N-methylolacrylamide, etc ,
- $\alpha,\beta$-ethylenically unsaturated monomers bearing a water-soluble polyoxyalkylene segment of the poly(ethylene oxide) type, such as poly(ethylene oxide) $\alpha$-methacrylates (Bisomer S20W, S10W, etc, from Laporte) or $\alpha,\omega$-dimethacrylates, Sipomer BEM from Rhodia ($\omega$-behenyl polyoxyethylene methacrylate), Sipomer SEM-25 from Rhodia ($\omega$-tristyrylphenyl polyoxyethylene methacrylate), etc ,
- $\alpha,\beta$-ethylenically unsaturated monomers which are precursors of hydrophilic units or segments, such as vinyl acetate, which, once polymerized, can be hydrolyzed in order to give rise to vinyl alcohol units or polyvinyl alcohol segments,
- vinylpyrrolidones,
- $\alpha,\beta$-ethylenically unsaturated monomers of the ureido type, and in particular 2-imidazolidinone-ethyl methacrylamide (Sipomer WAM II from Rhodia).

Examples of zwitterionic monomers (from which zwitterionic units can derive) include.

- sulfobetaine monomers, such as sulfopropyl dimethylammonium ethyl methacrylate (SPE from Raschig), sulfopropyldimethylammonium propylmethacrylamide (SPP from Raschig), and sulfopropyl-2-vinylpyridinium (SPV from Raschig),
- phosphobetaine monomers, such as phosphatoethyl trimethylammonium ethyl methacrylate,
- carboxybetaine monomers.

The copolymers can be prepared by conventional radical polymerization, or by any other polymerization process

Especially preferred units C, from the lists above or not, include units deriving from

- MAPTAC, which is [3-(Methacryloylamino)propyl]trimethylammonium chloride
- MAPTA-MES, which is [3-(Methacryloylamino)propyl]trimethylammonium Methyl sulfate
- DADMAC, which is Diallyldimethylammonium chloride
- DIQUAT, which is Methacryloamidopropyl-petamethyl-1,3-propylene-2-ol-amonium dichloride
- ADAMQUAT, which is [2-(Acryloyloxy)ethyl]trimethylammonium (chloride, Methyl sulfate or other version)
- MADAMQUAT, which is [2-(Methacryloyloxy)ethyl]trimethylammonium (chloride, Methyl sulfate or other version)
- DMAEMA, which is which is N-[3-(Dimethylamino)ethyl]methacrylamide, or
- DMAPMA, which is N-[3-(Dimethylamino)propyl]methacrylamide.

Especially preferred units C, from the lists above or not, include units deriving from

- Acrylic acid (AA), methacrylic acid,

- vinyl sulphonate,
- styrene sulphonate, or
- AMPS, which is 2-acrylamide-2-methyl-1-propanesulfonic acid

Especially preferred hydrophilic units N, from the lists above or not, include units deriving from.

- acrylamide (AM), or
- methacrylamide

Especially preferred hydrophobic units N, from the lists above or not, include units deriving from

- styrene,
- alkyl acrylates or methacrylates, wherein the alkyl group is for example a $C_1$-$C_{30}$ alkyl group

Especially preferred units Z, from the lists above or not, include

- carboxybetaine units, carboxypyrrolidinium units,
- sulfobétaïne units, sulfopyrrolidinium units,
- phosphonobetaine units, or phosphonopyrrolidinium units

Especially preferred polymers are the following

- a polymer deriving from MAPTAC, AA, and optionally AM,
- a polymer deriving from DADMAC, AA, and optionally AM, the molar ratio between DADMAC and AA being preferably of higher than 50/50, preferably of higher or equal to 60/40, the molar ratio between (DADMAC and AA) and AM (if any), being preferably of higher than 67/33, or
- a polymer deriving from DIQUAT, AA, and optionally AM, the molar ratio between DIQUAT and AA being preferably of from 20 to 80 to 80/20, the molar ratio between (DIQUAT and AA) and AM (if any), being preferably of higher than 67/33

Useful amphoteric or zwitterionic polymers are for example marketed by Rhodia in a form of solutions comprising the polymer and acidic or basic fillers, as Mirapol Surf S 100, 110, 200, 210, 400, 410, 411, 500, 510 Other useful polymers also include Polyquart 149 marketed by Cognis

Composition

The composition comprising the polymer can be in different forms The form usually depends upon the use of the composition. Thus the composition can be a fluid such as liquid, a gel, a foaming liquid The composition can also be in a solid form to be rendered fluid in situ upon contact with a wet surface and/or with a wet application mean

Liquid composition can be dispensed for example by spraying, by pouring or applying with a liquid dispensing device such as rollers or pumps Such devices are application means

The compositions can be diluted prior to being used, or during use Dilution is preferably performed with water. Dilution rates usually depend upon the use of the composition Furthermore dilutions approaches can depend upon the very consumer that uses the composition For compositions that are to be diluted, dilution rates can be of from 5 to 2000, preferably 5 to 500 for example

Where the composition is a liquid composition, for example a liquid concentrate composition, said composition can comprise a liquid medium, preferably an aqueous medium, an alcoholic medium, or a hydroxy-alcoholic medium. The medium can be a part of the composition, a part of a concentrate composition, or a part of a diluted composition, for example the diluting medium.

Further ingredients

The composition will usually comprise further ingredients of home-care, institutional-cleaning, or body-cleaning compositions. The further ingredients usually depend upon the use, destination and/or form of the composition These further ingredients are known by the one skilled in the art of preparing compositions comprising several ingredients (or formulation) to serve a use or a market

Thus the composition can comprise

- a carrier medium being an aqueous, alcoholic or hydroxyalcoolic medium,
- the polymer,
- optionally a surfactant, being an anionic surfactant, a non ionic surfactant, an amphoteric surfactant, a cationic surfactant, or mixture thereof,
- optionally further ingredients

Thus, the composition can comprise at least one surfactant. Compositions that comprise a surfactant are for example useful in compositions that are to provide cleaning or degreasing, such as hard-surface cleaning compositions, or personal cleansing products. Examples include liquid cleaning compositions, wnidow cleaners composition, kitchen, bathroom or toilet cleaning compositions, shower rinse compositions, make-up removal compositions, cleansing compositions, greasy skin-care compositions

Useful surfactants include anionic, non ionic, cationic, amphoteric (including zwitterionic) surfactants and mixtures thereof.

Anionic Surfactants

Anionic surfactants useful in the present invention are preferably selected from the group consisting of, linear alkylbenzene sulfonates, alpha olefin sulfonates, paraffin sulfonates, methyl ester sulfonates, alkyl sulfates, alkyl alkoxy sulfates, alkyl sulfonates, alkyl alkoxy carboxylate, alkyl alkoxylated sulfates, sarcosinates, taurinates, and mixtures thereof.

One type of anionic surfactant which can be utilized encompasses alkyl ester sulfonates. These are desirable because they can be made with renewable, nonpetroleum resources Preparation of the alkyl ester sulfonates surfactants components can be effected according to known methods disclosed in the technical literature For instance, linear esters of $C_8$-$C_{20}$ carboxylic acids can be sulfonated with gaseous $SO_3$ according to "The Journal of the American Oil Chemists Society," 52 (1975), pp 323-329 Suitable starting materials would include natural fatty substances as derived from tallow, palm, and coconut oils, etc

The preferred alkyl ester sulfonate surfactant, especially for laundry applications, comprises alkyl ester sulfonate surfactants of the structural formula

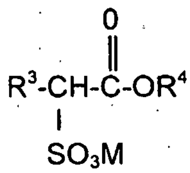

wherein $R^3$ is a $C_8$-$C_{20}$ hydrocarbyl, preferably an alkyl, or combination thereof, $R^4$ is a $C_1$-$C_6$ hydrocarbyl, preferably an alkyl, or combination thereof, and M is a soluble salt-forming cation Suitable salts include metal salts such as sodium, potassium, and lithium salts, and substituted or unsubstituted ammonium salts, such as methyl-, dimethyl, - trimethyl, and quaternary ammonium cations, e.g tetramethyl-ammonium and dimethyl piperdinium, and cations derived from alkanolamines, e g monoethanol-amine, diethanolamine, and triethanolamine.

Preferably, $R^3$ is $C_{10}$-$C_{16}$ alkyl, and $R^4$ is methyl, ethyl or isopropyl. Especially preferred are the methyl ester sulfonates wherein $R^3$ is $C_{14}$-$C_{16}$ alkyl

Alkyl sulfate surfactants are another type of anionic surfactant of importance for use herein In addition to providing excellent overall cleaning ability when used in combination with polyhydroxy fatty acid amides (see below), including good grease/oil cleaning over a wide range of temperatures, wash concentrations, and wash times, dissolution of alkyl sulfates can be obtained, as well as improved formulability in liquid detergent formulations are water soluble salts or acids of the formula $ROSO_3M$ wherein R preferably is a $C_{10}$-$C_{24}$ hydrocarbyl, preferably an alkyl or hydroxyalkyl having a $C_{10}$-$C_{20}$ alkyl component, more preferably a $C_{12}$-$C_{18}$ alkyl or hydroxyalkyl, and M is H or a cation, e g , an alkali or alkaline (Group IA or Group IIA) metal cation (e g , sodium, potassium, lithium, magnesium, calcium), substituted or unsubstituted ammonium cations such as methyl-, dimethyl and trimethyl ammonium and quaternary ammonium cations, e g , tetramethylammonium and dimethyl piperdinium, and cations denved from alkanolamines such as ethanolamine, diethanolamine, triethanolamine, and mixtures thereof, and the like Typically, alkyl chains of $C_{12}$-$C_{16}$ are preferred for lower wash temperatures (e g , below about 50°C) and $C_{16}$-$C_{18}$ alkyl chains are preferred for higher wash temperatures (e g , above about 50°C) Examples of these surfactants include: surfactants sold by Rhodia under the Rhodapan Trade Name

Alkyl alkoxylated sulfate surfactants are another category of useful anionic surfactant. These surfactants are water soluble salts or acids typically of the formula $RO(A)_mSO_3M$ wherein R is an unsubstituted $C_{10}$-$C_{24}$ alkyl or hy-

droxyalkyl group having a $C_{10}$-$C_{24}$ alkyl component, preferably a $C_{12}$-$C_{20}$ alkyl or hydroxyalkyl, more preferably $C_{12}$-$C_{18}$ alkyl or hydroxyalkyl, A is an ethoxy or propoxy unit, m is greater than zero, typically between about 0.5 and about 6, more preferably between about 0.5 and about 3, and M is H or a cation which can be for example, a metal cation (e.g , sodium, potassium, lithium, calcium, magnesium, etc.), ammonium or substituted-ammonium cation. Alkyl ethoxylated sulfates as well as alkyl propoxylated sulfates are contemplated herein Specific examples of substituted ammonium cations include methyl-, dimethyl-, trimethyl-ammonium and quaternary ammonium cations, such as tetramethyl-ammonium, dimethyl piperidinium and cations derived from alkanolamines, e.g. monoethanolamine, diethanolamine, and triethanolamine, and mixtures thereof Exemplary surfactants are $C_{12}$-$C_{18}$ alkyl polyethoxylate (1 0) sulfate, $C_{12}$-$C_{18}$ alkyl polyethoxylate (2 25) sulfate, $C_{12}$-$C_{18}$ alkyl polyethoxylate (3 0) sulfate, and $C_{12}$-$C_{18}$ alkyl polyethoxylate (4.0) sulfate wherein M is conveniently selected from sodium and potassium. Surfactants for use herein can be made from natural or synthetic alcohol feedstocks Chain lengths represent average hydrocarbon distributions, including branching Examples of these surfactants include surfactants sold by Rhodia under the Rhodapex Trade Name

Other Anionic Surfactants - Other anionic surfactants useful for detersive purposes can also be included in the compositions hereof These can include salts (including, for example, sodium, potassium, ammonium, and substituted ammonium salts such as mono-, di- and triethanolamine salts) of soap, $C_8$-$C_{20}$ linear alkylbenzenesulphonates, for example sold by Rhodia under the Rhodacal trande name, $C_8$-$C_{22}$ primary or secondary alkanesulphonates, $C_8$-$C_{24}$ olefinsulphonates, sulphonated polycarboxylic acids prepared by sulphonation of the pyrolyzed product of alkaline earth metal citrates, e g., as described in British patent specification No. 1,082,179, alkyl glycerol sulfonates, fatty acyl glycerol sulfonates, fatty oleyl glycerol sulfates, alkyl phenol ethylene oxide ether sulfates, paraffin sulfonates, alkyl phosphates, isothionates such as the acyl isothionates, N-acyl taurates, fatty acid amides of methyl tauride, alkyl succinamates and sulfosuccinates, monoesters of sulfosuccinate, for example sold by Rhodia under the Geropon trade name (especially saturated and unsaturated $C_{12}$-$C_{18}$ monoesters) diesters of sulfosuccinate (especially saturated and unsaturated $C_6$-$C_{14}$ diesters), N-acyl-sarcosinates, sulfates of alkylpolysaccharides such as the sulfates of alkylpolyglucoside (the nonionic nonsulfated compounds being described below), branched primary alkyl sulfates, alkyl polyethoxy carboxylates such as those of the formula $RO(CH_2CH_2O)_kCH_2COO^-M+$ wherein R is a $C_8$-$C_{22}$ alkyl, k is an integer from 0 to 10, and M is a soluble salt-forming cation, and fatty acids esterified with isethionic acid and neutralized with sodium hydroxide. Resin acids and hydrogenated resin acids are also suitable, such as rosin, hydrogenated rosin, and resin acids and hydrogenated resin acids present in or derived from tall oil

Further examples are given in "Surface Active Agents and Detergents" (Vol. I and II by Schwartz, Perry and Berch) A variety of such surfactants are also generally disclosed in U S. Patent 3,929,678, issued December 30, 1975 to Laughlin, et al at Column 23, line 58 through Column 29, line 23

Nonionic Surfactants

Suitable nonionic detergent surfactants are generally disclosed in U S Patent 3,929,678, Laughlin et al., issued December 30, 1975, at column 13, line 14 through column 16, line 6, incorporated herein by reference Exemplary, non-limiting classes of useful nonionic surfactants include: alkyl dialkyl amine oxide, for example sold by Rhodia under the Rhodamox trade name, alkyl ethoxylate, for example sold by Rhodia under the Rhodasurf trade name, alkanoyl glucose amide, alkyl betaines, for example sold by Rhodia under the Mirataine trade name, and mixtures thereof Other nonionic surfactants for use herein include

The polyethylene, polypropylene, and polybutylene oxide condensates of alkyl phenols. In general, the polyethylene oxide condensates are preferred. These compounds include the condensation products of alkyl phenols having an alkyl group containing from about 6 to about 12 carbon atoms in either a straight chain or branched chain configuration with the alkylene oxide. In a preferred embodiment, the ethylene oxide is present in a amount equal to from about 5 to about 25 moles of ethylene oxide per mole of alkyle phenol. Commercially available nonionic surfactants of this type include surfactants sold by Rhodia under the Igepal trade name. These are commonly referred to as phenol alkoxylates, (e.g., alkyl phenol ethoxylates).

The condensation products of aliphatic alcohols with from about 1 to about 25 moles of ethylene oxide. The alkyl chain of the aliphatic alcohol can either be straight or branched, primary or secondary, and generally contains from about 8 to about 22 carbon atoms. Particularly preferred are the condensation products of alcohols having an alkyl group containing from about 10 to about 20 carbon atoms with from about 2 to about 18 moles of ethylene oxide per mole of alcohol.

Examples of commercially available nonionic surfactants of this type include TergitolB 15-S-9 (the condensation product of $C_{11}$-$C_{15}$ linear secondary alcohol with 9 moles ethylene oxide), Tergitol 24-L-6 NMW (the condensation product of $C_{12}$-$C_{14}$ primary alcohol with 6 moles ethylene oxide with a narrow molecular weight distribution), both marketed by Union Carbide Corporation; Neodol® 45-9 (the condensation product of $C_{14}$-$C_{15}$ linear alcohol with 9 moles of ethylene oxide), Neodol® 23-6.5 (the condensation product of $C_{12}$-$C_{13}$ linear alcohol with 6.5 moles of ethylene oxide), Neodol® 45-7 (the condensation product of $C_{14}$-$C_{15}$ linear alcohol with 7 moles of ethylene oxide), Neodol® 45-4 (the condensation

product of $C_{14}$-$C_{15}$ linear alcohol with 4 moles of ethylene oxide), marketed by Shell Chemical Company, Rhodasurf IT, DB, and B marketed by Rhodia, Plurafac LF 403, marketed by BASF, and Kyro® EOB (the condensation product of $C_{13}$-$C_{15}$ alcohol with 9 moles ethylene oxide), marketed by The Procter & Gamble Company. Other commercially available nonionic surfactants include Dobanol 91-8® marketed by Shell Chemical Co. and Genapol UD-080® marketed by Hoechst. This category of nonionic surfactant is referred to generally as "alkyl ethoxylates."

The condensation products of ethylene oxide with a hydrophobic base formed by the condensation of propylene oxide with propylene glycol. The hydrophobic portion of these compounds preferably has a molecular weight of from about 1500 to about 1800 and exhibits water insolubility. The addition of polyoxyethylene moieties to this hydrophobic portion tends to increase the water solubility of the molecule as a whole, and the liquid character of the product is retained up to the point where the polyoxyethylene content is about 50% of the total weight of the condensation product, which corresponds to condensation with up to about 40 moles of ethylene oxide Examples of compounds of this type include: certain of the commercially-available Pluronic® surfactants, marketed by BASF, and Antarox, marketed by Rhodia.

The condensation products of ethylene oxide with the product resulting from the reaction of propylene oxide and ethylenediamine The hydrophobic moiety of these products consists of the reaction product of ethylenediamine and excess propylene oxide, and generally has a molecular weight of from about 2500 to about 3000 This hydrophobic moiety is condensed with ethylene oxide to the extent that the condensation product contains from about 40% to about 80% by weight of polyoxyethylene and has a molecular weight of from about 5,000 to about 11,000. Examples of this type of nonionic surfactant include certain of the commercially available TetronicB compounds, marketed by BASF

Semi-polar nonionic surfactants are a special category of nonionic surfactants which include water-soluble amine oxides containing one alkyl moiety of from about 10 to about 18 carbon atoms and 2 moieties selected from the group consisting of alkyl groups and hydroxyalkyl groups containing from about 1 to about 3 carbon atoms, water-soluble phosphine oxides containing one alkyl moiety of from about 10 to about 18 carbon atoms and 2 moieties selected from the group consisting of alkyl groups and hydroxyalkyl groups containing from about 1 to about 3 carbon atoms; and water-soluble sulfoxides containing one alkyl moiety of from about 10 to about 18 carbon atoms and a moiety selected from the group consisting of alkyl and hydroxyalkyl moieties of from about 1 to about 3 carbon atoms

Semi-polar nonionic detergent surfactants include the amine oxide surfactants having the formula

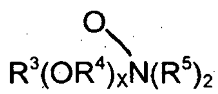

wherein $R^3$ is an alkyl, hydroxyalkyl, or alkyl phenyl group or mixtures thereof containing from about 8 to about 22 carbon atoms, $R^4$ is an alkylene or hydroxyalkylene group containing from about 2 to about 3 carbon atoms or mixtures thereof, x is from 0 to about 3, and each $R^5$ is an alkyl or hydroxyalkyl group containing from about 1 to about 3 carbon atoms or a polyethylene oxide group containing from about 1 to about 3 ethylene oxide groups' The $R^5$ groups can be attached to each other, e g , through an oxygen or nitrogen atom, to form a ring structure These amine oxide surfactants in particular include $C_{10}$-$C_{18}$ alkyl dimethyl amine oxides and $C_8$-$C_{12}$ alkoxy ethyl dihydroxy ethyl amine oxides.

Alkylpolysaccharides disclosed in U S Patent 4,565,647, Llenado, issued January 21, 1986, having a hydrophobic group containing from about 6 to about 30 carbon atoms, preferably from about 10 to about 16 carbon atoms and a polysaccharide, e g., a polyglycoside, hydrophilic group containing from about 1.3 to about 10, preferably from about 1 3 to about 3, most preferably from about 1 3 to about 2 7 saccharide units Any reducing saccharide containing 5 or 6 carbon atoms can be used, e g , glucose, galactose and galactosyl moieties can be substituted for the glucosyl moieties. (Optionally the hydrophobic group is attached at the 2-, 3-, 4-, etc positions thus giving a glucose or galactose as opposed to a glucoside or galactoside.) The intersaccharide bonds can be, e g , between the one position of the additional saccharide units and the 2-, 3-, 4-, and/or 6- positions on the preceding sacchande units

Optionally, and less desirably, there can be a polyalkylene-oxide chain joining the hydrophobic moiety and the polysaccharide moiety. The preferred alkyleneoxide is ethylene oxide. Typical hydrophobic groups include alkyl groups, either saturated or unsaturated, branched or unbranched containing from about 8 to about 18, preferably from about 10 to about 16, carbon atoms Preferably, the alkyl group is a straight chain saturated alkyl group The alkyl group can contain up to about 3 hydroxy groups and/or the polyalkyleneoxide chain can contain up to about 10, preferably less than 5, alkyleneoxide moieties. Suitable alkyl polysaccharides are octyl, nonyl, decyl, undecyldodecyl, tridecyl, tetradecyl, pentadecyl, hexadecyl, heptadecyl, and octadecyl, di-, tri-, tetra-, penta-, and hexaglucosides, galactosides, lactosides, glucoses, fructosides, fructoses and/or galactoses Suitable mixtures include coconut alkyl, di-, tri-, tetra-, and pentaglucosides and tallow alkyl tetra-, penta-, and hexa-glucosides

The preferred alkylpolyglycosides have the formula:

$$R^2O(C_nH_{2n}O)_t(glycosyl)_x$$

wherein $R^2$ is selected from the group consisting of alkyl, alkyl-phenyl, hydroxyalkyl, hydroxyalkylphenyl, and mixtures thereof in which the alkyl groups contain from about 10 to about 18, preferably from about 12 to about 14, carbon atoms, n is 2 or 3, preferably 2, t is from 0 to about 10, preferably 0, and x is from about 1 3 to about 10, preferably from about 1 3 to about 3, most preferably from about 1.3 to about 2.7. The glycosyl is preferably derived from glucose To prepare these compounds, the alcohol or alkylpolyethoxy alcohol is formed first and then reacted with glucose, or a source of glucose, to form the glucoside (attachment at the I-position). The additional glycosyl units can then be attached between their 1 position and the preceding glycosyl units 2-, 3-, 4- and/or 6-position, preferably predominantly the 2-position.

Non ionic surfactant include fatty acid amide surfactants having the formula

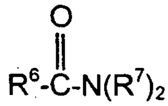

wherein $R^6$ is an alkyl group containing from about 7 to about 21 (preferably from about 9 to about 17) carbon atoms and each $R^7$ is selected from the group consisting of hydrogen, $C_1$-$C_4$ alkyl, $C_1$-$C_4$ hydroxyalkyl, and $-(C^2H_4O)_xH$ where x varies from about 1 to about 3. Preferred amides are $C_8$-$C_{20}$ ammonia amides, monoethanolamides, diethanolamides, and isopropanolamydes.

Non ionic surfactant include also derivates of terpenic compounds, for example NOPOL alkoxylates, such as NOPOL ethoxylates, propoxylates, radom or blocky ethopropolylates. Such compounds are marketed by Rhodia as Rhodoclean range

Cationic Surfactants

Cationic detersive surfactants can also be included in detergent compositions of the present invention. Cationic surfactants include the ammonium surfactants such as alkyldimethylammonium halogenides, and those surfactants having the formula: $[R^2(OR^3)_y][R^4(OR^3)_y]_2R^5N^+X^-$ wherein $R^2$ is an alkyl or alkyl benzyl group having from about 8 to about 18 carbon atoms in the alkyl chain, each $R^3$ is selected from the group consisting of $-CH_2CH_2-$, $-CH_2CH(CH_3)-$, $-CH_2CH(CH_2OH)-$, $-CH_2CH_2CH_2-$, and mixtures thereof; each $R^4$ is selected from the group consisting of $C_1$-$C_4$ alkyl, $C_1$-$C_4$ hydroxyalkyl, benzyl, ring structures formed by joining the two $R^4$ groups, $-CH_2CHOHCHOHCOR^6CHOH-CH_2OH$ wherein $R^6$ is any hexose or hexose polymer having a molecular weight less than about 1000, and hydrogen when y is not 0; $R^5$ is the same as $R^4$ or is an alkyl chain wherein the total number of carbon atoms of $R^2$ plus $R^5$ is not more than about 18, each y is from 0 to about 10 and the sum of the y values is from 0 to about 15, and X is any compatible anion

Other cationic surfactants useful herein are also described in U.S Patens 4,228,044, Cambre, issued October 14, 1980, incorporated herein by reference

Other Surfactants

Ampholytic surfactants can be incorporated into the detergent compositions hereof. These surfactants can be broadly described as aliphatic derivatives of secondary or tertiary amines, or aliphatic derivatives of heterocyclic secondary and tertiary amines in which the aliphatic radical can be straight chain or branched. One of the aliphatic substituents contains at least about 8 carbon atoms, typically from about 8 to about 18 carbon atoms, and at least one contains an anionic water-solubilizing group, e g., carboxy, sulfonate, sulfate. See U S. Patent No 3,929,678 to Laughlin et al., issued December 30, 1975 at column 19, lines 18-35 for examples of ampholytic surfactants Preferred amphoteric include $C_{12}$-$C_{18}$ alkyl ethoxylates ("AE") including the so-called narrow peaked alkyl ethoxylates and $C_6$-$C_{12}$ alkyl phenol alkoxylates (especially ethoxylates and mixed ethoxy/propoxy), $C_{12}$-$C_{18}$ betaines and sulfobetaines ("sultaines"), $C_{10}$-$C_{18}$ amine oxides, and mixtures thereof

Zwitterionic surfactants can also be incorporated into the detergent compositions hereof These surfactants can be broadly described as derivatives of secondary and tertiary amines, derivatives of heterocyclic secondary and tertiary amines, or derivatives of quaternary ammonium, quaternary phosphonium or tertiary sulfonium compounds See U.S Patent No 3,929,678 to Laughlin et al., issued December 30, 1975 at column 19, line 38 through column 22, line 48 for examples of zwitterionic surfactants Ampholytic and zwitterionic surfactants are generally used in combination with one or more anionic and/or nonionic surfactants

Usefull amphoteric surfactants include especially amphoacetates or diamphoacaetates, and bétaïnes (alkylbetaines and alkylamidoalkylbetaines).

Other optional ingredients of the composition include:

- thickening polymers,

- hydrophilizing polymers (the function of hydrophilizing can be also delivered by the ampholytic or zwitterionic polymer),
- deposition agents or deposition aid agents, for example deposition polymers, such as silicones,
- anti-foaming agents,
- foaming agents,
- foam stabilizing and/or enhancing agents,
- perfumes or fragrances,
- builders (detergency adjuvants), including organic builders and inorganic builders,
- buffers,
- salts or fillers,
- chelatants,
- colorants,
- preservatives, ,
- enzymes,
- corrosion inhibitors,
- scale inhibitors,
- dyes or pigments,
- optical bighteners,
- soiling suspending agents,
- freezing-thawing stabilizers,
- solvents,
- opacifiers,
- pearlescence agents

The composition can include from 0 0001 % by weight of the polymer to 5% by weight The composition when applied (in a diluted form, or neat) has advantageously from 0,005 to 2 %, preferably 0 01 to 0 5% by weight of the polymer
The composition can be prepared conventionally by mixing the ingredients thereof It can be further diluted by the final user

Process of removing dirt

The process comprises the steps of:

a) Applying fluid composition comprising an ampholytic or zwitterionic polymer onto the surface having dirt or make-up, by pouring or spraying the composition onto the surface or by using an application mean,
b) simultaneously or subsequently spreading and/or wiping the composition onto the surface, with a wiping or spreading fibrous or porous mean, and then,
c) optionally removing the liquid, or a part thereof, form the surface with a drying fibrous or porous mean,

some dirt or make-up being transferred from the surface to the application mean and/or the wiping mean and/or the spreading mean, and/or the drying mean
The dirt to be removed and transferred to the typically comprises mineral particles such as

- carbon black,
- carbon oxides,
- clays,
- pigments of a make-up composition or
- mixtures thereof.

The dirt can also, additionally or alternatively, include geasy soils, sebum, or food
The surface can be

- glass, for example comprised in windows,
- tile or ceramic, for example comprised in kitchens, bathrooms, toilet bowls, showers, floors, or teeth
- metal, for example comprised in window frames, or floors,
- plastic, for example comprised in windows, furniture, or floors, especially in kitchen or bathroom furniture or panels, for example melamine, formica, linoleum,
- wood or leather, optionally waxed, for example comprised in furniture or floor,

- concrete, optionally waxed , for example comprised in floors,
- the leave of a plant, or
- a keratinous surface such as skin or hair

In windows, floors, furniture or any other hard surface, the composition can be applied by any conventional mean, including direct spraying, spraying after dilution, application with using vehicles such as mops, pads, wipes, cloths, sponges, papers

Step a) application step

Step a) is an application step of the composition The composition is in a fluid form, and has been optionally diluted before.

In one embodiment Step a) is performed by pouring the composition onto the surface. Pouring can be performed from a recipient where the composition has been diluted such as a bucket, or from a dispensing bottle comprising the composition The route from a dilution recipient is often used in dilutable cleaner and/or so called "all purpose cleaners". The route from a dispensing bottle is often used in kitchen or bathroom cleaners. The dispensing bottle can be provided with appropriate means such as a pump or a mean that provides a jet upon applying a pressure on the bottle

In another embodiment the composition is sprayed onto the surface. This is often used in kitchen, bathroom or windows cleaners. This is also used in floor cleaning devices provided with both a spraying nozzle and wiping or spreading fibrous or porous pads or wipes

In another embodiment, the composition is applied, in a diluted or concentrate form, with an application mean such as a fibrous or porous mean, for example a sponge, a mop, a cloth, a fabric, a paper, a wipe Thus the composition can be poured or spayed onto the application mean as described above The application mean can be also impregnated completely with the composition, for example by immerging in the composition This route is for example used when using "wet wipes" or when immerging a sponge, cloth, or mop into a recipient such as a bucket or a sink The wipe is also referred to as a support of the composition Then the application mean is applied onto the surface or is generally used as a wiping and/or spreading mean in step b)

In a particular embodiment, the composition is formed in situ by contacting a fibrous or porous support comprising the polymer onto a wet surface, or by contacting a fibrous or porous support comprising the polymer with water before applying said mean to the surface In this embodiment the support and application mean can be a dry wipe.

Step b) wiping or/and spreading step

Step b) is a wiping or spreading step Wiping and/or spreading is the action of providing the composition on a larger area than the application area or than the application mean area, usually by moving a mean (wiping and/or spreading mean) up and down and/or right and left, and/or forward and backward Upon applying some pressure the wiping and/or spreading can also be referred to as rubbing and/or scraping.

If step a) has been performed with an application mean such as a fibrous or porous mean, then step b) is usually indeed performed simultaneously, with the same mean (the wiping and/or spreading mean is the application mean).

If step b) has been preformed by pouring or spraying the composition onto the surface, then step b) is usually performed subsequently.

The wiping and/or spreading means can typically be a wipe, a cloth, a pad, a mop, a fabric or a paper

Step c) drying step

Step c) is a dying step In this optional step possible excess of liquid is removed. This can be performed by letting the surface drying (no action), by heating, or by applying a substantially dry fibrous or porous mean onto the surface, such an absorbing paper, a fabric or sponge, or a wipe Indeed some drying can occur simultaneously to wiping and/or spreading

Transfer of the dirt or make-up from the surface can happen during one or several of steps a) to c), to the application mean and/or the wiping and/or spreading mean, and/or drying mean

Advantageously

- the application mean and/or the wiping mean and/or the spreading mean, and/or the drying mean has a dirt or make-up transfer index DeltaE of higher then 2, preferably of higher than 5, and/or
- the surface has a dirt or make-up transfer index DeltaE of higher then 2, preferably of higher than 5

According to the invention, the application mean and/or the wiping mean and/or the spreading mean, and/or the drying mean is preferably visually dirtier after step b) or optional step c) then before. It is also preferred that the dirt or make-up transfer be higher than the dirt or make-up transfer than the dirt transfer obtained with the same process with a composition that does not comprise the ampholytic or zwitterionic polymer.

Process means and fibrous or porous means

The application mean and/or the wiping mean and/or the spreading mean, and/or the drying mean, as mentioned above, can be for example

- a wipe
- a cloth
- a fabric
- a pad, for example a cotton pad
- a mop,
- a sponge,
- a fibrous puff, for example a cotton puff,
- a cotton bud, or
- an absorbing paper

These mean are known from the one skilled in the art, and the literature comprise numerous descriptions of such means

Fibrous means, such as wipes, are for examples non woven fabrics comprising fibers such as fibers of polyester, cellulose or cellulose denvatives (both being understood as cellulosic materials), polypropylene, and mixtures compnsing such fibers

Porous means include sponges

Advantageously the application mean and/or the wiping mean and/or the spreading mean, and/or the drying mean, as mentioned above comprises a cellulosic material. It is for example

- a wipe, a cloth, a pad, a mop, an absorbing parer or a fabric comprising cellulosic fibers, or
- a cellulosic or partly cellulosic sponge.

The application mean can be a support, supporting the composition, such as a wipe. The application mean can be the wiping mean and/or the spreading mean, and/or the drying mean, such as a wipe, a sponge, a mop, a pad, an absorbing paper or a cloth. It is mentioned that step a), step b) and optional step c) can be performed simultaneously with the same means and by a same wiping movement

In one particular embodiment step a) comprises spraying the composition onto a surface, and step b) comprises wiping the surface with a fibrous or porous mean, for example a wipe, cloth, pad, or mop

In one particular embodiment, the composition is a liquid cleaning composition, being applied onto the surface in a neat form or diluted form, being optionally diluted or further diluted in situ during application step a) or spreading or wiping step b)

In another particular embodiment, the composition is a liquid composition supported on a fibrous or porous support, said support being the application mean and/or the spreading or wiping mean, for example a wet wipe.

In another particular embodiment, the liquid composition is formed in situ when contacting with the surface being wet, a dry fibrous ou porous support comprising the polymer, said support being the application mean and/or the spreading or wiping mean, for example a dry wipe.

In another particular embodiment, the liquid composition is a non dilutable cleaner, such as a window cleaner or a toilet-bowl cleaner, being applied onto the surface in a neat form, being optionally diluted or further diluted in situ during application step a) or spreading or wiping step b)

The example below which is not limitative presents some further details or advantages of the invention.

Examples

Standard wipes (viscose) from Eurowipes are impregnated with a kitchen cleaning type formulation

Kitchen cleaner formulation

- Rhodoclean EFC 1% (Rhodia)
- Mirataine H2CHA: 0 9% (diamphoacetate surfactant, Rhodia)
- IPA: 3%
- Dowanol PNB 3% (Solvent, Dow Chemical)
- Mirapol Surf S 200 3% or 0% (solution comprising an ampholytic polymer, Rhodia)
- NaOH to adjust pH about 8
- Water (pure) to 100%

Protocol for impregnation and drying.

Impregnation

Wipes are impregnated in a beaker, with the tested formulation (2g of solution for 1g of wipe). Then, they are lightly wrung (no drop) and stored in closed airtight plastic bag in waiting the drying

Wipes are stored in closed airtight plastic bag directly until application

Then, performances are evaluated visually:

The surfaces are wiped with the wipe (humid) Surfaces for floor are the "natural floor" Surfaces for kitchen are the white ceramic tile treated with a kitchen soil Figure 1 is a picture after wiping of the wipe impregnated with a comparative composition that does not comprise Mirapol Surf S 200

Figure 2 is a picture after wiping of the wipe impregnated with the composition that comprises that Mirapol Surf S 200

There is a visible "dirt pick up" with the wipes containing the ampholytic polymer

## Claims

1. The use, as an agent for transferring dirt or make-up from surfaces to application means and/or wiping means and/or spreading means and/or drying means, said means comprising a cellulosic material, of an ampholytic or zwitterionic polymer, in a process for removing dirt or make-up from surfaces, comprising the steps of

   a) Applying a fluid composition comprising an ampholytic or zwitterionic polymer onto the surface having dirt or make-up, by pouring or spraying the composition onto the surface or by using an application mean,
   b) simultaneously or subsequently spreading and/or wiping the composition onto the surface, with a wiping or spreading fibrous or porous mean, and then,
   c) optionally removing the liquid, or a part thereof, from the surface with a drying fibrous or porous mean,

   some dirt or make-up being transferred from the surface to the application mean and/or the wiping mean and/or the spreading mean, and/or the drying mean.

2. A use according to claim 1, wherein the surface is:

   - a domestic or institutional hard surface,
   - a tooth,
   - a keratinous surface such as skin or hair, or
   - a plant.

3. A use according any of the preceding claims, wherein:

   - the application mean and/or the wiping mean and/or the spreading mean, and/or the drying mean has a dirt or make-up transfer index DeltaE of higher then 2, preferably of higher than 5, and/or
   - the surface has a dirt or make-up transfer index DeltaE of higher then 2, preferably of higher than 5,

   wherein the transfer index Delta E is

$$DeltaE = \sqrt{DL^2 + Da^2 + Db^2}$$

wherein:

DL = L before the process - L after the process
Da = a before the process - a after the process
Db = b before the process - b after the process

L, a and b being the CIE L*a*b* coordinates.

**4.** A use according to any of the preceding claims, wherein the application mean and/or the wiping mean and/or the spreading mean, and/or the drying mean is visually dirtier after step b) or optional step c) then before.

**5.** A use according any of the preceding claims, wherein the dirt or make-up transfer is higher than the dirt or make-up transfer than the dirt transfer obtained with the same process with a composition that does not comprise the ampholytic or zwitterionic polymer.

**6.** A use according any of the preceding claims, wherein the dirt being transferred comprises:

- mineral particles such as:
  - carbon black,
  - carbon oxides,
  - clays,
  - pigments of a make-up composition, or
  - mixtures thereof, and/or
- greasy soils, sebum or food.

**7.** A use according to any of the preceding claims, wherein the surface is:

- glass, for example comprised in windows,
- tile or ceramic, for example comprised in kitchens, bathrooms, toilet bowls, showers, teeth,
- metal, for example comprised in window frames,
- plastic, such as formica, or linoleum, for example comprised in floors, windows, or furniture, especially in kitchen or bathroom furniture or panels,
- wood or leather for example comprised in furniture,
- the leave of a plant, or
- a keratinous surface such as skin or hair.

**8.** A use according to any of the preceding claims, wherein the application mean and/or the wiping mean and/or the spreading mean, and/or the drying mean, comprises cellulosic material.

**9.** A use according to claim 8, wherein the application mean and/or the wiping and/or the spreading mean, and/or the drying mean, is

- a wipe, a cloth, a pad, a mop, an absorbing paper, or a fabric comprising cellulosic fi bers, or
- a cellulosic or partly cellulosique sponge.

**10.** A use according to any of claims 1 to 8, wherein the application mean is a support, supporting the composition, such as a wipe.

**11.** A use according to any of the preceding claims, wherein the application mean is the wiping mean and/or the spreading mean, and/or the drying mean, such as a wipe, a sponge, a mop, a pad, an absorbing paper or a cloth.

**12.** A use according to any the preceding claims wherein step a), step b) and optional step c) are performed simultaneously with the same means and by a same wiping movement.

**13.** A use according to any of claims 1 to 9, wherein step a) comprises spraying the composition onto a surface, and step b) comprises wiping the surface with a fibrous or porous mean, for example a wipe, cloth, pad, or mop.

**14.** A use according to any of the preceding claims, wherein the polymer is an ampholytic polymer comprising:

- cationic or potentially cationic units C,
- anionic or potentially anionic units A, and
- optionally non ionic, hydrophilic or hydrophobic units N.

**15.** A use according to the preceding claim, wherein the cationic or potentially cationic units are units deriving from cationic or potentially cationic monomers selected from the group consisting of:

- MAPTAC, MAPTA-MES,
- DADMAC,
- DIQUAT,
- ADAMQUAT, MADAMQUAT,
- DMAEMA, and
- DMAPMA.

**16.** A use according to any of claims 14 or 15, wherein the anionic or potentially anionic units are units deriving from anionic or potentially anionic monomers selected from the group consisting of:

- Acrylic acid, methacrylic acid,
- vinyl sulphonate,
- styrene sulphonate, and
- AMPS.

**17.** A use according to any of claims 14 to 16, wherein the non ionic hydrophilic units are units deriving from non ionic hydrophilic monomers selected from the group consisting of:

- acrylamide, methacrylamide.

**18.** A use according to any of claims 14 to 17, wherein the non ionic hydrophobic units are units deriving from non ionic hydrophobic monomers selected from the group consisting of:

- styrene, and
- alkyl acrylates or methacrylates.

**19.** A use according to any of claims 14 to 17, wherein the polymer is:

- a polymer deriving from MAPTAC, AA, and optionally AM,
- a polymer deriving from DADMAC; AA, and optionally AM, the molar ratio between DADMAC and AA being preferably of higher than 50/50, the molar ratio between (DADMAC and AA) and AM if any, being preferably of higher than 67/33, or
- a polymer deriving from DIQUAT, AA, and optionally AM, the molar ratio between DIQUAT and AA being preferably of from 20 to 80 to 80/20, the molar ratio between (DIQUAT and AA) and AM if any, being preferably of higher than 67/33.

**20.** A use according to any of claims 1 to 13, wherein the polymer is a zwitterionic polymer comprising:

a') zwitterionic units bearing a cationic charge and an anionic charge on the same unit,
b') optionally other units, being anionic or potentially anionic units, or non ionic hydrophilic or hydrophobic units.

**21.** A use according to claim 20, wherein the zwitterionic units are

- carboxybetaine units, carboxypyrrolidinium units,
- sulfobétaïne units, sulfopyrrolidinium units,
- phosphonobetaine units, or phosphonopyrrolidinium units.

**22.** A use according to any of the preceding claims, wherein the composition comprises:

- a carrier medium being an aqueous, alcoholic or hydroxyalcoolic medium,
- the polymer,
- optionally a surfactant, being an anionic surfactant, a non ionic surfactant, an amphoteric surfactant, a cationic surfactant, or mixture thereof,
- optionally further ingredients.

**23.** A use according to one of the preceding claims, wherein the composition is a liquid cleaning composition, being applied onto the surface in a neat form or diluted form, being optionally diluted or further diluted in situ during application step a) or spreading or wiping step b).

**24.** A use according to one of claims 1 to 22, wherein the composition is a liquid composition supported on a fibrous or porous support, said support being the application mean and/or the spreading or wiping mean, for example a wet wipe.

**25.** A use according to one of claims 1 to 22 wherein the liquid composition is formed in situ when contacting with the surface being wet, a dry fibrous ou porous support comprising the polymer, said support being the application mean and/or the spreading or wiping mean, for example a dry wipe.

**26.** A use according to one of claims 1 to 22 wherein the liquid composition is a non dilutable cleaner, such as a window cleaner or a toilet-bowl cleaner, being applied onto the surface in a neat form, being optionally diluted or further diluted in situ during application step a) or spreading or wiping step b).

**Patentansprüche**

**1.** Verwendung eines ampholytischen oder zwitterionischen Polymers als Mittel zum Überführen von Schmutz oder Make-up von Oberflächen auf Applikationsmittel und/oder Wischmittel und/oder Auftragungsmittel und/oder Trocknungsmittel, wobei das Mittel ein cellulosisches Material umfasst, bei einem Verfahren zum Entfernen von Schmutz oder Make-up von Oberflächen, umfassend die Schritte

a) Aufbringen einer Fluidzusammensetzung, die ein ampholytisches oder zwitterionisches Polymer umfasst, auf die Oberfläche, die Schmutz oder Make-up aufweist, durch Gießen oder Sprühen der Zusammensetzung auf die Oberfläche oder unter Verwendung eines Applikationsmittels,
b) gleichzeitig oder anschließend mit einem faserigen oder porösen Wisch- oder Auftragungsmittel Auftragen und/oder Wischen der Zusammensetzung auf die Oberfläche und anschließend
c) gegebenenfalls Entfernen der Flüssigkeit oder eines Teils davon von der Oberfläche mithilfe eines faserigen oder porösen Trocknungsmittels,

wobei etwas Schmutz oder Make-up von der Oberfläche auf das Applikationsmittel und/oder das Wischmittel und/oder das Auftragungsmittel und/oder das Trocknungsmittel übertragen wird.

**2.** Verwendung gemäß Anspruch 1, wobei die Oberfläche

- eine häusliche oder institutionelle harte Oberfläche,
- ein Zahn,
- eine keratinöse Oberfläche, wie z. B. Haut oder Haar, oder
- eine Pflanze

ist.

**3.** Verwendung gemäß einem der vorstehenden Ansprüche, wobei:

- das Applikationsmittel und/oder das Wischmittel und/oder das Auftragungsmittel und/oder das Trocknungsmittel einen Schmutz- oder Make-up-Übertragungsindex DeltaE von größer als 2 aufweist, vorzugsweise von größer als 5, und/oder
- die Oberfläche einen Schmutz- oder Make-up-Übertragungsindex DeltaE von größer als 2 aufweist, vorzugsweise von größer als 5,

wobei der Übertragungsindex DeltaE

$$DeltaE = \sqrt{DL^2 + Da^2 + Db^2}$$

ist, wobei:

DL = L vor dem Verfahren - L nach dem Verfahren
Da = a vor dem Verfahren - a nach dem Verfahren
Db = b vor dem Verfahren - b nach dem Verfahren

L, a und b die CIE L*a*b-Koordinaten sind.

**4.** Verwendung gemäß einem der vorstehenden Ansprüche, wobei das Applikationsmittel und/oder das Wischmittel und/oder das Auftragungsmittel und/oder das Trocknungsmittel nach Schritt b) oder dem optionalen Schritt c) visuell schmutziger ist als davor.

**5.** Verwendung gemäß einem der vorstehenden Ansprüche, wobei die Schmutz- oder Make-up-Übertragung höher ist als die Schmutz- oder Make-up-Übertragung, die mit dem gleichen Verfahren mit einer Zusammensetzung erhalten wird, die das ampholytische oder zwitterionische Polymer nicht umfasst.

**6.** Verwendung gemäß einem der vorstehenden Ansprüche, wobei der übertragene Schmutz:

- mineralische Partikel, wie z. B.
  - Ruß,
  - Kohleoxide,
  - Tone
  - Pigmente einer Make-up-Zusammensetzung oder
  - Gemische davon und/oder
- fettigen Schmutz, Talg oder Lebensmittel umfasst.

**7.** Verwendung gemäß einem der vorstehenden Ansprüche, wobei die Oberfläche:

- Glas, beispielsweise in Fenstern enthalten,
- Fliesen oder Keramik, beispielsweise in Küchen, Badezimmern, Toilettenbecken, Duschen, Zähnen enthalten,
- Metall, beispielsweise in Fensterrahmen enthalten,
- Kunststoff, wie z. B. Resopal oder Linoleum, beispielsweise in Fußböden, Fenstern oder Möbeln enthalten, insbesondere in Küchen- oder Badezimmermöbeln oder Täfelungen,
- Holz oder Leder, beispielsweise in Möbeln enthalten,
- das Blatt einer Pflanze oder
- eine keratinöse Oberfläche, wie z. B. Haut oder Haar,

ist.

**8.** Verwendung gemäß einem der vorstehenden Ansprüche, wobei das Applikationsmittel und/oder das Wischmittel und/oder das Auftragungsmittel und/oder das Trocknungsmittel cellulosisches Material umfasst.

**9.** Verwendung gemäß Anspruch 8, wobei das Applikationsmittel und/oder das Wischmittel und/oder das Auftragungsmittel und/oder das Trocknungsmittel

- ein Wischtuch, ein Tuch, ein Kissen, ein Mopp, ein absorbierendes Papier oder ein Gewebe, das cellulosische Fasern umfasst, oder
- ein cellulosischer oder teilweise cellulosischer Schwamm

ist.

**10.** Verwendung gemäß einem der Ansprüche 1 bis 8, wobei das Applikationsmittel ein Träger ist, der die Zusammensetzung trägt, wie z. B. ein Wischtuch.

**11.** Verwendung gemäß einem der vorstehenden Ansprüche, wobei das Applikationsmittel das Wischmittel und/oder das Auftragungsmittel und/oder das Trocknungsmittel ist, wie z. B. ein Wischtuch, ein Schwamm, ein Mopp, ein Kissen, ein absorbierendes Papier oder ein Tuch.

**12.** Verwendung gemäß einem der vorstehenden Ansprüche, wobei Schritt a), Schritt b) und der optionale Schritt c) gleichzeitig mit dem gleichen Mittel und mit der gleichen Wischbewegung durchgeführt werden.

**13.** Verwendung gemäß einem der Ansprüche 1 bis 9, wobei Schritt a) Sprühen der Zusammensetzung auf eine Oberfläche umfasst und Schritt b) Wischen der Oberfläche mit einem faserigen oder porösen Mittel, wie z. B. einem Wischtuch, Tuch, Kissen oder Mopp, umfasst.

**14.** Verwendung gemäß einem der vorstehenden Ansprüche, wobei das Polymer ein ampholytisches Polymer ist, umfassend:

- kationische oder potenziell kationische Einheiten C,
- anionische oder potenziell anionische Einheiten A und
- gegebenenfalls nichtionische, hydrophile oder hydrophobe Einheiten N.

**15.** Verwendung gemäß einem der vorstehenden Ansprüche, wobei die kationischen oder potenziell kationischen Einheiten Einheiten sind, die abgeleitet sind von kationischen oder potenziell kationischen Monomeren ausgewählt aus der Gruppe bestehend aus:

- MAPTAC, MAPTA-MES,
- DADMAC,
- DIQUAT,
- ADAMQUAT, MADAMQUAT,
- DMAEMA und
- DMAPMA.

**16.** Verwendung gemäß einem der Ansprüche 14 oder 15, wobei die anionischen oder potenziell anionischen Einheiten Einheiten sind, die abgeleitet sind von anionischen oder potenziell anionischen Monomeren ausgewählt aus der Gruppe bestehend aus:

- Acrylsäure, Methacrylsäure,
- Vinylsulfonat,
- Styrolsulfonat und
- AMPS.

**17.** Verwendung gemäß einem der Ansprüche 14 bis 16, wobei die nichtionischen hydrophilen Einheiten Einheiten sind, die abgeleitet sind von nichtionischen hydrophilen Monomeren ausgewählt aus der Gruppe bestehend aus:

- Acrylamid, Methacrylamid.

**18.** Verwendung gemäß einem der Ansprüche 14 bis 17, wobei die nichtionischen hydrophoben Einheiten Einheiten sind, die abgeleitet sind von nichtionischen hydrophoben Monomeren ausgewählt aus der Gruppe bestehend aus:

- Styrol und
- Alkylacrylaten oder -methacrylaten.

**19.** Verwendung gemäß einem der Ansprüche 14 bis 17, wobei das Polymer:

- ein Polymer, abgeleitet von MAPTAC, AA und gegebenenfalls AM,
- ein Polymer, abgeleitet von DADMAC, AA und gegebenenfalls AM, wobei das Molverhältnis zwischen DADMAC und AA vorzugsweise höher als 50/50 ist, das Molverhältnis zwischen (DADMAC und AA) und AM, falls vorhanden, vorzugsweise höher als 67/33 ist, oder

- ein Polymer, abgeleitet von DIQUAT, AA und gegebenenfalls AM, wobei das Molverhältnis zwischen DIQUAT und AA vorzugsweise von 20 zu 80 bis 80/20 beträgt, das Molverhältnis zwischen (DIQUAT und AA) und AM, falls vorhanden, vorzugsweise höher als 67/33 ist,

ist.

**20.** Verwendung gemäß einem der Ansprüche 1 bis 13, wobei das Polymer ein zwitterionisches Polymer ist, umfassend:

a') zwitterionische Einheiten, die eine kationische Ladung und eine anionische Ladung an der gleichen Einheit tragen,
b') gegebenenfalls andere Einheiten, die anionische oder potenziell anionische Einheiten sind, oder nichtionische hydrophile oder hydrophobe Einheiten.

**21.** Verwendung gemäß Anspruch 20, wobei die zwitterionischen Einheiten

- Carboxybetaineinheiten, Carboxypyrrolidiniumeinheiten,
- Sulfobetaineinheiten, Sulfopyrrolidiniumeinheiten,
- Phosphonobetaineinheiten oder Phosphonopyrrolidiniumeinheiten

sind.

**22.** Verwendung gemäß einem der vorstehenden Ansprüche, wobei die Zusammensetzung umfasst:

- ein Trägermedium, das ein wässriges, alkoholisches oder hydroxyalkoholisches Medium ist,
- das Polymer,
- gegebenenfalls ein grenzflächenaktives Mittel, das ein anionisches grenzflächenaktives Mittel, ein nichtionisches grenzflächenaktives Mittel, ein amphoteres grenzflächenaktives Mittel, ein kationisches grenzflächenaktives Mittel oder ein Gemisch davon ist,
- gegebenenfalls weitere Inhaltsstoffe.

**23.** Verwendung gemäß einem der vorstehenden Ansprüche, wobei die Zusammensetzung eine flüssige Reinigungszusammensetzung ist, die in einer reinen Form oder einer verdünnten Form, die gegebenenfalls während des Schritts des Aufbringens a) oder des Schritts des Auftragens oder Wischens b) *in situ* verdünnt oder weiterverdünnt wird, auf die Oberfläche aufgebracht wird.

**24.** Verwendung gemäß einem der Ansprüche 1 bis 22, wobei die Zusammensetzung eine flüssige Zusammensetzung ist, die auf einem faserigen oder porösen Träger getragen ist, wobei der Träger das Applikationsmittel und/oder das Auftragungsmittel oder Wischmittel, beispielsweise ein feuchtes Wischtuch, ist.

**25.** Verwendung gemäß einem der Ansprüche 1 bis 22, wobei die flüssige Zusammensetzung *in situ* gebildet wird, wenn die Oberfläche, die feucht ist, in Kontakt mit einem trockenen faserigen oder porösen Träger kommt, der das Polymer umfasst, wobei der Träger das Applikationsmittel und/oder das Auftragungsmittel oder Wischmittel, beispielsweise ein trockenes Wischtuch, ist.

**26.** Verwendung gemäß einem der Ansprüche 1 bis 22, wobei die flüssige Zusammensetzung ein nichtverdünnbares Reinigungsmittel, ist, wie z. B. ein Fensterreinigungsmittel oder ein Toilettenbecken-Reinigungsmittel, das in einer reinen Form auf die Oberfläche aufgebracht wird und gegebenenfalls während des Schritts des Aufbringens a) oder des Schritts des Auftragens oder Wischens b), *in situ* verdünnt oder weiterverdünnt wird.

## Revendications

**1.** Utilisation, comme agent pour le transfert de saletés ou de maquillage de surfaces à un moyen d'application et/ou un moyen d'essuyage et/ou un moyen d'étalement et/ou un moyen de séchage, ledit moyen comprenant un matériau cellulosique, d'un polymère ampholytique ou zwittérionique, dans un procédé d'élimination de saletés ou de maquillage de surfaces, comprenant les étapes qui consistent à :

a) appliquer une composition fluide comprenant un polymère ampholytique ou zwittérionique sur la surface

comportant les saletés ou le maquillage, en versant ou en pulvérisant la composition sur la surface ou en utilisant un moyen d'application,
b) étaler et/ou essuyer simultanément ou consécutivement la composition sur la surface, avec un moyen d'essuyage ou d'étalement fibreux ou poreux, puis
c) optionnellement éliminer le liquide, ou une partie de celui-ci, de la surface avec un moyen de séchage fibreux ou poreux,

des saletés ou du maquillage étant transférés de la surface au moyen d'application et/ou au moyen d'essuyage et/ou au moyen d'étalement et/ou au moyen de séchage.

**2.** Utilisation selon la revendication 1, dans laquelle la surface est :

- une surface dure située dans un lieu d'habitation ou dans un établissement,
- une dent,
- une surface kératineuse telle que la peau ou les cheveux, ou
- une plante.

**3.** Utilisation selon l'une quelconque des revendications précédentes, dans laquelle :

- le moyen d'application et/ou le moyen d'essuyage et/ou le moyen d'étalement et/ou le moyen, de séchage a un indice de transfert de saletés ou de maquillage DeltaE supérieur à 2, préférablement supérieur à 5, et/ou
- la surface a un indice de transfert de saletés ou de maquillage DeltaE supérieur à 2, préférablement supérieur à 5,

l'indice de transfert DeltaE étant :

$$DeltaE = \sqrt{DL^2 + Da^2 + Db^2}$$

où :

DL = L avant le procédé - L après le procédé
Da = a avant le procédé - a après le procédé
Db = b avant le procédé - b après le procédé
L, a et b étant les coordonnées CIE L*a*b*.

**4.** Utilisation selon l'une quelconque des revendications précédentes, dans laquelle le moyen d'application et/ou le moyen d'essuyage et/ou le moyen d'étalement et/ou le moyen de séchage est visuellement plus sale après l'étape_ b) ou l'étape optionnelle c) qu'avant celle-ci.

**5.** Utilisation selon l'une quelconque des revendications précédentes, dans laquelle le transfert des saletés ou du maquillage est supérieur au transfert des saletés ou du maquillage obtenu avec le même procédé en utilisant une composition qui ne contient pas le polymère ampholytique ou zwittérionique.

**6.** Utilisation selon l'une quelconque des revendications précédentes, dans laquelle les saletés transférées comprennent :

- des particules minérales telles que :

  - le noir de carbone,
  - les oxydes de carbone,
  - les argiles,
  - des pigments d'une composition de maquillage, ou
  - des mélanges de ceux-ci, et/ou

- des salissures grasses, du sébum ou des aliments.

7. Utilisation selon l'une quelconque des revendications précédentes, dans laquelle la surface est :

   - du verre, présent par exemple dans des fenêtres,
   - des carreaux ou de la céramique, présents par exemple dans des cuisines, des salles de bains, des cuvettes de toilettes, des cabines de douche, des dents,
   - un métal, présent par exemple dans des encadrements de fenêtres,
   - des matières plastiques, telles que le formica ou le linoléum, présentes par exemple dans des sols, des fenêtres ou des meubles, en particulier dans des meubles ou des panneaux de cuisine ou de salle de bains,
   - du bois ou du cuir, présent par exemple dans des meubles,
   - la feuille d'une plante, ou
   - une surface kératineuse telle que la peau ou les cheveux.

8. Utilisation selon l'une quelconque des revendications précédentes, dans laquelle le moyen d'application et/ou le moyen d'essuyage et/ou le moyen d'étalement et/ou le moyen de séchage comprend un matériau cellulosique.

9. Utilisation selon la revendication 8, dans laquelle le moyen d'application et/ou le moyen d'essuyage et/ou le moyen d'étalement et/ou le moyen de séchage est

   - une lingette, un chiffon, un tampon, un balai-éponge, un papier absorbant, ou un tissu comprenant des fibres cellulosiques, ou
   - une éponge cellulosique ou partiellement cellulosique.

10. Utilisation selon l'une quelconque des revendications 1 à 8, dans laquelle le moyen d'application est un support, portant la composition, tel qu'une lingette.

11. Utilisation selon l'une quelconque des revendications précédentes, dans laquelle le moyen d'application est le moyen d'essuyage et/ou le moyen d'étalement et/ou le moyen de séchage, tel qu'une lingette, une éponge, un balai-éponge, un tampon, un papier absorbant ou un chiffon.

12. Utilisation selon l'une quelconque des revendications précédentes, dans laquelle l'étape a), l'étape b) et l'étape optionnelle c) sont exécutées simultanément avec le même moyen et par un même mouvement d'essuyage.

13. Utilisation selon l'une quelconque des revendications 1 à 9, dans laquelle l'étape a) comprend la pulvérisation de la composition sur une surface, et l'étape b) comprend l'essuyage de la surface avec un moyen fibreux ou poreux, par exemple une lingette, un chiffon, un tampon, ou un balai-éponge.

14. Utilisation selon l'une quelconque des revendications précédentes, dans laquelle le polymère est un polymère ampholytique comprenant :

   - des unités cationiques ou potentiellement cationiques C,
   - des unités anioniques ou potentiellement anioniques A, et
   - optionnellement des unités non ioniques, hydrophiles ou hydrophobes N.

15. Utilisation selon la revendication précédente, dans laquelle les unités cationiques ou potentiellement cationiques sont des unités dérivées de monomères cationiques ou potentiellement cationiques sélectionnés dans le groupe constitué :

   - du MAPTAC, du MAPTA-MES,
   - du DADMAC,
   - du DIQUAT,
   - de l'ADAMQUAT, du MADAMQUAT,
   - du DMAEMA, et
   - du DMAPMA.

16. Utilisation selon l'une quelconque des revendications 14 ou 15, dans laquelle les unités anioniques ou potentiellement anioniques sont des unités dérivées de monomères anioniques ou potentiellement anioniques sélectionnés dans le groupe constitué :

- de l'acide acrylique, de l'acide méthacrylique,
- du sulfonate de vinyle,
- du sulfonate de styrène, et
- de l'AMPS.

**17.** Utilisation selon l'une quelconque des revendications 14 à 16, dans laquelle les unités non ioniques hydrophiles sont des unités dérivées de monomères non ioniques hydrophiles sélectionnés dans le groupe constitué:

- de l'acrylamide, du méthacrylamide.

**18.** Utilisation selon l'une quelconque des revendications 14 à 17, dans laquelle les unités non ioniques hydrophobes sont des unités dérivées de monomères non ioniques hydrophobes sélectionnés dans le groupe constitué :

- du styrène, et
- d'acrylates ou de méthacrylates d'alkyles.

**19.** Utilisation selon l'une quelconque des revendications 14 à 17, dans laquelle le polymère est :

- un polymère dérivé de MAPTAC, d'AA, et optionnellement d'AM,
- un polymère dérivé de DADMAC, d'AA, et optionnellement d'AM, le rapport molaire entre le DADMAC et l'AA étant préférablement supérieur à 50/50, le rapport molaire entre (le DADMAC et l'AA) et l'AM, le cas échéant, étant préférablement supérieur à 67/33, ou
- un polymère dérivé de DIQUAT, d'AA, et éventuellement d'AM, le rapport molaire entre le DIQUAT et l'AA étant préférablement de 20 à 80 à 80/20, le rapport molaire entre (le DIQUAT et l'AA) et l'AM, le cas échéant, étant préférablement supérieur à 67/33.

**20.** Utilisation selon l'une quelconque des revendications 1 à 13, dans laquelle le polymère est un polymère zwittérionique comprenant :

a') des unités zwittérioniques portant une charge cationique et une charge anionique sur la même unité,
b') optionnellement d'autres unités, lesquelles sont des unités anioniques ou potentiellement anioniques, ou non ioniques hydrophiles ou hydrophobes.

**21.** Utilisation selon la revendication 20, dans laquelle les unités zwittérioniques sont :

- des unités de carboxybétaïne, des unités de carboxypyrrolidinium,
- des unités de sulfobétaïne, des unités de sulfopyrrolidinium,
- des unités de phosphonobétaïne, ou des unités de phosphonopyrrolidinium.

**22.** Utilisation selon l'une quelconque des revendications précédentes, dans laquelle la composition comprend :

- un milieu de support, lequel est un milieu aqueux, alcoolique ou hydroxy-alcoolique,
- le polymère,
- optionnellement un tensioactif, lequel est un tensioactif anionique, un tensioactif non ionique, un tensioactif amphotère, un tensioactif cationique, ou un mélange de ceux-ci,
- optionnellement des ingrédients supplémentaires.

**23.** Utilisation selon l'une des revendications précédentes, la composition étant une composition de nettoyage liquide, laquelle est appliquée sur la surface telle quelle ou sous une forme diluée, en pouvant optionnellement être diluée ou diluée davantage sur place durant l'étape d'application a) ou l'étape d'étalement ou d'essuyage b).

**24.** Utilisation selon l'une des revendications 1 à 22, la composition étant une composition liquide portée sur un support fibreux ou poreux, ledit support étant le moyen d'application et/ou le moyen d'étalement ou d'essuyage, par exemple une lingette humide.

**25.** Utilisation selon l'une des revendications 1 à 22, dans laquelle la composition liquide est formée sur place au moment de l'entrée en contact avec la surface en cours de mouillage, un support sec fibreux ou poreux comprenant le polymère, ledit support étant le moyen d'application et/ou le moyen d'étalement ou d'essuyage, par exemple une

lingette sèche.

**26.** Utilisation selon l'une des revendications 1 à 22, dans laquelle la composition liquide est un produit de nettoyage non diluable, tel qu'un produit de nettoyage pour fenêtres ou un produit de nettoyage pour cuvettes de toilettes, lequel est appliqué sur la surface tel quel, en pouvant optionnellement être dilué ou dilué davantage sur place durant l'étape d'application a) ou l'étape d'étalement ou d'essuyage b).

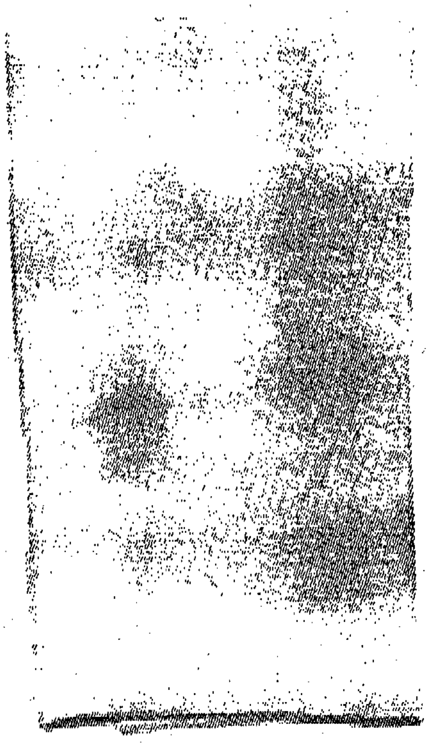

Figure 1

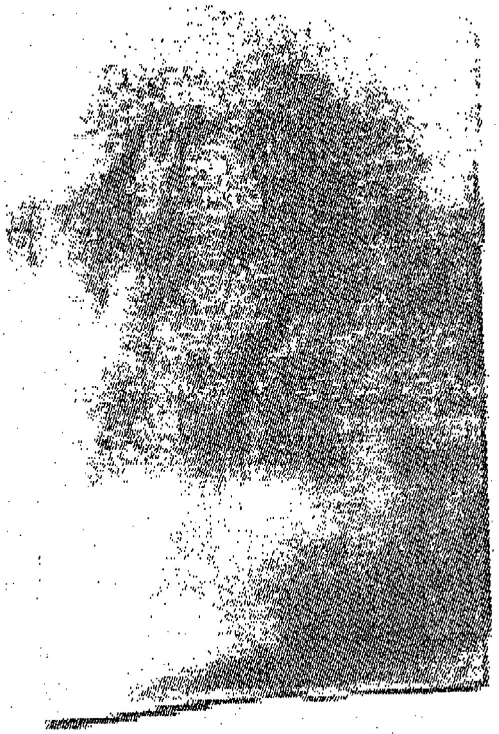

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4784789 A
- GB 1082179 A
- US 3929678 A, Laughlin
- US 4565647 A, Llenado
- US 4228044 A, Cambre


### Non-patent literature cited in the description


- *The Journal of the American Oil Chemists Society,* 1975, vol. 52, 323-329